(19) Europäisches Patentamt
European Patent Office
Office européen des brevets

(11) Veröffentlichungsnummer: **0 070 444**
**B1**

(12) # EUROPÄISCHE PATENTSCHRIFT

(45) Veröffentlichungstag der Patentschrift: **19.09.84**

(21) Anmeldenummer: **82105970.6**

(22) Anmeldetag: **05.07.82**

(51) Int. Cl.³: **C 07 H 15/04**, C 07 H 17/04, C 07 H 15/18, C 07 H 1/00, C 07 C 41/50

(54) **Verfahren zur Herstellung von 1,1'-Diacetalen.**

(30) Priorität: **17.07.81 DE 3128271**

(43) Veröffentlichungstag der Anmeldung: **26.01.83 Patentblatt 83/4**

(45) Bekanntmachung des Hinweises auf die Patenterteilung: **19.09.84 Patentblatt 84/38**

(84) Benannte Vertragsstaaten: **AT BE CH DE FR GB IT LI NL SE**

(56) Entgegenhaltungen:
**TETRAHEDRON LETTERS, Band 21, 1980, Seiten 1357-1358, Pergamon Press Ltd., (GB);**
**TETRAHEDRON LETTERS, Band 22, Nr. 34, 1981, Seiten 3239-3242, (GB);**
**ANGEW. CHEM. INT. ED. ENGL., Band 20, Nr. 11, 1981, Seite 969, Verlag Chemie GmbH, Weinheim (DE);**

(73) Patentinhaber: **BAYER AG, Konzernverwaltung RP Patentabteilung, D-5090 Leverkusen 1 Bayerwerk (DE)**

(72) Erfinder: **Tietze, Lutz-Friedjan, Prof. Dr., Goerlitzer Strasse 72, D-3400 Goettingen (DE)**
Erfinder: **Fischer, Roland, Danziger Strasse 38b, D-3412 Noertenhardenberg (DE)**

**EP 0 070 444 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die stereoselektive Bildung von Glycosiden gehört zu den anspruchsvollsten Synthese-Problemen in der Naturstoffchemie (G. Wulff und G. Röhle, Angew. Chem. 86, 173 [1974]; Angew. Chem. Int. Ed. Engl. 13, 157 [1974]). Besonders schwierig ist hierbei der Aufbau von Glycosiden mit 1,1'-Diacetalstruktur, wie sie in den 1,1-verknüpften Disacchariden oder Iridoidglycosiden (L.-F. Tietze, U. Niemeyer, Chem. Ber. 111, 2423 [1978]; L.-F. Tietze, U. Niemeyer, P. Marx, K.-H. Glüsenkamp, L. Schwenen, Tetrahedron 36, 735 [1980]; L.-F. Tietze, U. Niemeyer, P. Marx, K.-H. Glüsenkamp, Tetrahedron 36, 1231 [1980]) vorliegen.

Es wurde nun eine überraschend einfache Methode zur Synthese von 1,1'-Diacetalen der allgemeinen Formel I gefunden, die sich u.a. auch zur stereoselektiven Synthese von Glycosiden mit 1,1'-Diacetalstruktur eignet.

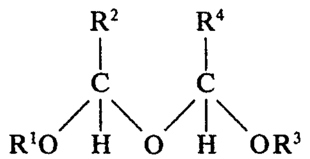

(I)

Verbindungen der Formel I erhält man, indem man die an sich bekannten Silylverbindungen der Struktur II (siehe u.a. L. Birkofer, A. Ritter, F. Bentz, Chem. Ber. 97, 2196 [1964]; A. Klemmer, E. Buhe, R. Kutz, Liebigs Ann. Chem. 739, 185 [1970])

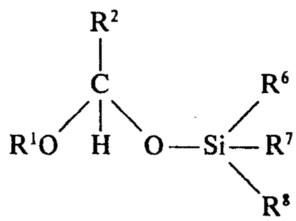

(II)

mit Acetalen der allgemeinen Formel III

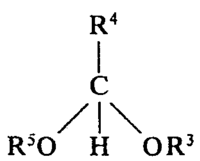

(III)

in Gegenwart eines Katalysators der Struktur IV oder V

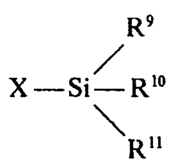

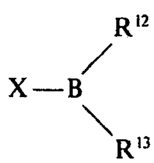

(IV) (V)

in einem geeigneten Lösungsmittel umsetzt.

In den vorstehend genannten allgemeinen Formeln bedeutet

$R_1$ und $R_2$ die gleich oder verschieden sein können, einen gegebenenfalls substituierten aliphatischen gesättigten oder ungesättigten Kohlenwasserstoffrest, vorzugsweise mit 1–6 C-Atomen und wobei als Substituenten 1–6, bzw. 1–3 Substituenten vorzugsweise aus der Gruppe (geschütztes) OH, O-Alkyl mit vorzugsweise 1–4 C-Atomen, Alkyloxycarbonyl mit vorzugsweise 1–4 C-Atomen oder Phenyl in Betracht kommen. $R_1$ und $R_2$ können miteinander verbunden sein.

$R_3$ gegebenenfalls substituiertes Alkyl vorzugsweise mit 1–6 C-Atomen

$R_4$ gegebenenfalls substituiertes O-Alkyl mit vorzugsweise 1–6 C-Atomen oder gegebenenfalls substituierter gesättigter oder ungesättigter aliphatischer Rest mit 1–6 C-Atomen wobei als Substituenten für $R_3$ und $R_4$ 1–6 vorzugsweise 1–3 Substituenten vorzugsweise

die für $R_1$ und $R_2$ genannten und Halogen in Betracht kommen. $R_3$ und $R_4$ können miteinander verbunden sein.

$R_5$ gegebenenfalls substituiertes Alkyl mit 1–10 C-Atomen, vorzugsweise 1–6 C-Atomen oder gegebenenfalls substituiertes Phenyl wobei als Substituenten vorzugsweise Halogen, Nitro oder O-Alkyl mit vorzugsweise 1–4 C-Atomen in Betracht kommen

$R_6$–$R_{13}$ die gleich oder verschieden sein können Alkyl mit 1–8 C-Atomen vorzugsweise Methyl.

$R_1$ und $R_2$ können bevorzugt zusammen einen Alkylenrest mit vorzugsweise 3–4 C-Atomen darstellen, der gegebenenfalls wie bei $R_1$ und $R_2$ angegeben substituiert sein kann.

X steht für $FSO_3$, $CF_3CO_2$, $ClO_4$, $BF_4$, $CH_3SO_3$ oder $CF_3(CF_2)_n-SO_3$ mit $n = 0–4$ vorzugsweise $CF_3SO_3$.

Besonders bevorzugt stellt

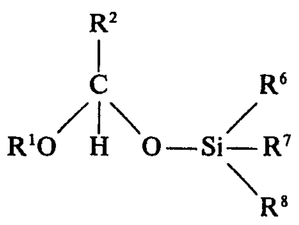

(II)

Verbindungen der allgemeinen Formel

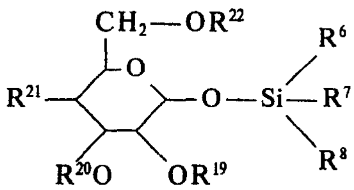

(VI)

dar, wobei $R^{19}$–$R^{22}$ gleich oder verschieden sein können und eine OH-Schutzgruppe darstellen.

Insbesondere liegen die Substituenten $OR^{19}$–$OR^{21}$ und $CH_2-O-R^{22}$ in VI in der gluco-Konfiguration vor.

Als OH-Schutzgruppen eignen sich die üblichen Schutzgruppen wie Acetyl, Benzyloxycarbonyl, Benzyl oder Trityl.

Zur Durchführung der Reaktion gibt man in einem geeigneten Lösungsmittel wie vorzugsweise $CH_2Cl_2$, $CHCl_3$, Acetonitril, Diethylether oder Tetrahydrofuran zu II äquimolare Mengen oder einen Überschuß III und etwa 1/10 Äquivalent des Katalysators IV oder V hinzu. Führt man die Reaktion in einem Temperaturbereich zwischen −80 und −40°C durch, erfolgt die Bildung von I mit Retention der Konfiguration an C-1, bei 0 bis 100°C erfolgt Inversion.

## Beispiele

Das folgende Formelschema verdeutlicht die nachstehend tabellarisch aufgeführten Umsetzungen. In der Beschreibung und in den Tabellen wird jeweils auf die Ziffern unter den Formeln Bezug genommen.

Edukt 1 Edukt 2 Produkt

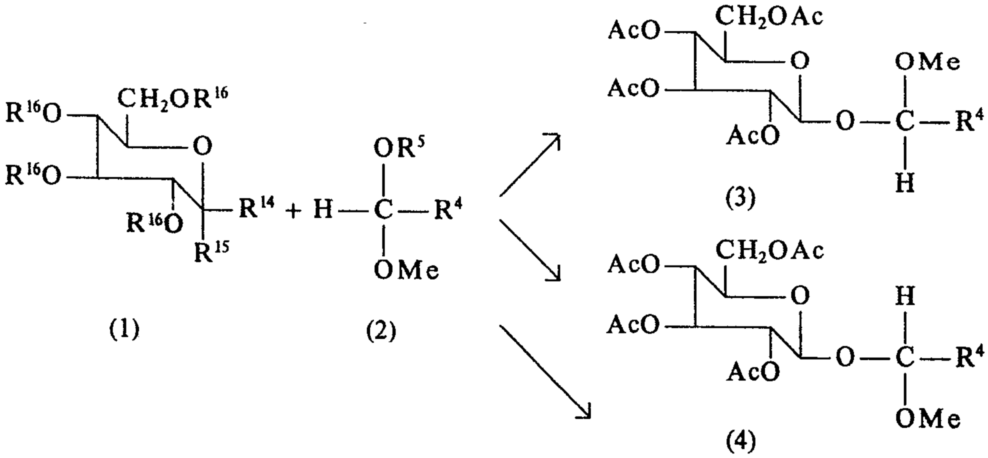

(1) (2) (3) (4)

Me = Methyl
Ac = Acetyl
Bn = Benzyl

$R^{16}$ = Ac, Bn
$R^{14}$ = H, $R^{15}$ = $OSi(CH_3)_3$ oder:
$R^{14}$ = $OSi(CH_3)_3$, $R^{15}$ = H

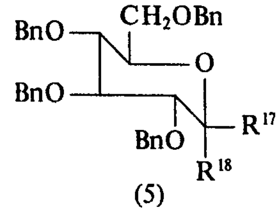

(5)

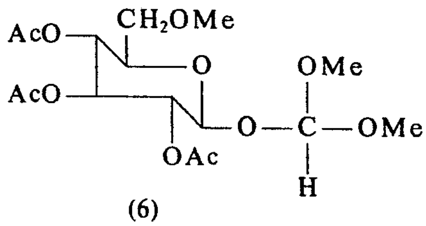

(6)

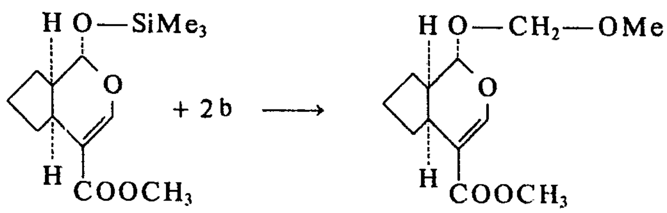


(7) (8)

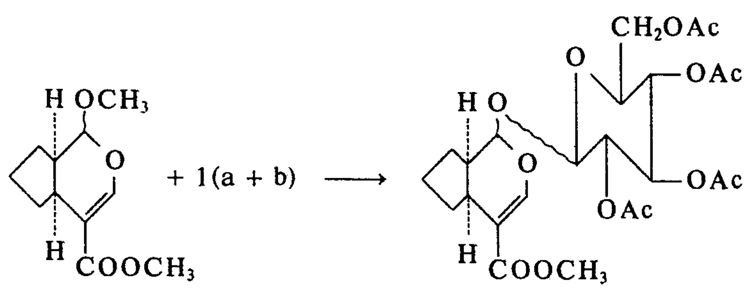


(9) (10)

Die zur Darstellung der Produkte (3 und 4) benötigten Acetale (2) können auch durch die jeweiligen Aldehyde (11) und Methyl-trimethylsilylether (12) ersetzt werden (vgl. T. Tsunoda, et al., Tetrahedron Letters 1980, 1357). Die hierbei erzielten Ausbeuten an (3 und 4) sind gleich oder besser als die in Tabelle 2 angegebenen.

$R-CHO$ (11) $CH_3-O-Si(CH_3)_3$ (12)

z. B. ist $R = CH_2Ph$, $n\text{-}C_3H_7$, $CH_2OMe$, $CH_2Cl$

Allgemeine Arbeitsvorschrift

Zu einer Lösung von 0,25 mmol Silylverbindung (1), 0,25 mmol Aldehyd (11) und 0,35 mmol Methyl-trimethylsilylether (12) in 2 ml wasserfreiem Dichlormethan gibt man bei −70°C unter einer Inertgas-Atmosphäre 0,2 ml einer 0,1 molaren Lösung von Trimethylsilyl-trifluormethansulfonat in Dichlormethan und rührt 20 bis 60 h bei dieser Temperatur. Der Reaktionsverlauf kann dünnschichtchromatographisch an Kieselgel (Laufmittel: Hexan/Essigester 1 : 1) verfolgt werden.

Es wurde weiterhin gefunden, daß die Zugabe von etwa 0,5 Moläquivalenten Aceton, bezogen auf z. B. Trimethylsilylglucose die Reaktionsgeschwindigkeit bei der Acetalisierung stark erhöhen kann.

| Edukt 1 | $R^{16}$ | $R^{14}$ | $R^{15}$ |
|---|---|---|---|
| 1 a | Ac | $OSiMe_3$ | H |
| 1 b | Ac | H | $OSiMe_3$ |
| 1 c | Bn | $OSiMe_3$ | H |
| 1 d | Bn | H | $OSiMe_3$ |

| Produkt | | $R^4$ |
|---|---|---|
| 3 a | 4 a | H |
| 3 b | 4 b | $CH_2Ph$ |
| 3 c | 4 c | $n\text{-}C_3H_7$ |
| 3 d | 4 d | $CH_2-CH(OMe)_2$ |
| 3 e | 4 e | $CH_2-OMe$ |
| 3 f | 4 f | $CH_2Cl$ |
| 3 g | 4 g | $CH_2Br$ |
| 3 h | | OMe |

| Produkt | $R^{17}$ | $R^{18}$ |
|---|---|---|
| 5 a | H | $OCH_2-OMe$ |
| 5 b | $OCH_2-OMe$ | H |

| Edukt 2 | $R^5$ | $R^4$ |
|---|---|---|
| 2 a | Me | H |
| 2 b | Ph | H |
| 2 c | Me | $CH_2Ph$ |
| 2 d | Me | $n\text{-}C_3H_7$ |
| 2 e | Me | $CH_2-CH(OMe)_2$ |
| 2 f | Me | $CH_2-OMe$ |
| 2 g | H | $CH_2Cl$ |
| 2 h | Me | $CH_2Br$ |
| 2 i | Me | OMe |

Die Umsetzungen erfolgten nach folgenden Arbeitsvorschriften

A) Zu einer Lösung von 0,25 mmol Silylverbindung (1 bzw. 7) und 0,50 mmol Acetal (2) in 3 ml wasserfreiem Dichlormethan gibt man bei −70°C unter einer Inertgas-Atmosphäre 0,2 ml einer 0,1 molaren Lösung von Trimethylsilyl-trifluormethansulfonat in Dichlormethan und rührt 20 bis 60 h bei dieser Temperatur. Der Reaktionsverlauf kann dünnschichtchromatographisch an Kieselgel (Laufmittel: Hexan/Essigester 1 : 1) verfolgt werden.

B) Zu einer Lösung von 0,25 mmol Silylverbindung (1) und 0,50 mmol Acetal (2) in 5 ml wasserfreiem Dichlormethan gibt man bei 0°C unter einer Inertgasatmosphäre 0,1 ml einer 0,1 molaren Lösung von Trimethylsilyl-trifluormethansulfonat in Dichlormethan und rührt 4 bis 16 h bei dieser Temperatur. Der Reaktionsverlauf kann dünnschichtchromatographisch an Kieselgel (Laufmittel: Hexan/Essigester 1 : 1) verfolgt werden.

C) Zu einer Lösung von 105 mg (0,25 mmol) Silylverbindung (1 a) und 53,0 mg (0,50 mmol) Methylorthoformiat (2 i) in 3 ml wasserfreiem Dichlormethan gibt man bei −30°C unter einer Inertgasatmosphäre 0,2 ml einer 0,1 molaren Lösung von Trimethylsilyltrifluormethansulfonat in Dichlormethan und rührt ca. 40 h bei dieser Temperatur. Nach Aufarbeitung erhält man 70 mg eines Gemisches aus (3 h) und (6).

D) Nach Methode C werden 42,4 (0,2 mmol) (9) mit 84,0 mg (0,2 mmol) (1) ($\alpha,\beta$-Gemisch) umgesetzt (Reaktionsdauer 6 Tage). Man erhält 93,8 mg (89 %) isomere Glucoside (10). Die Identifizierung erfolgte durch Vergleich mit Originalsubstanzen.

## Aufarbeitung

Zur Aufarbeitung versetzt man mit 0,1 ml Triethylamin, wäscht die Reaktionsmischung mit gesättigter $NaHCO_3$- und NaCl-Lösung und trocknet über $Na_2SO_4/Na_2CO_3$ (1 : 1). Nach Abdampfen des Lösungsmittels im Vakuum erhält man ein nahezu einheitliches Produkt, das zur weiteren Reinigung an Kieselgel chromatographiert werden kann.

Z) Zur Bestimmung der Konfiguration an C-1 wurden die Tetraacetate (3 und 4) solvolytisch mit Methanol/Natriummethanolat in die freien Glucoside übergeführt.

Die nachfolgende Tabelle 2 zeigt die eingesetzten Edukte, die Verfahrensweise, Ausbeuten sowie Produkte und ihre Charakterisierung.

Tabelle 2

| Edukte | Methode | Produkt | Ausbeute (%) | $^1$H—NMR ($\delta = 0$ ppm) | IR | Analyse Ber. | Analyse Gef. |
|---|---|---|---|---|---|---|---|
| 1 a + 2 a | A | 1-O-(1'-Methoxy-methyl)-2,3,4,6-tetra-O-acetyl-β-D-glucopyranose (3a) | 75 | (100 MHz, $CDCl_3$): 1.97 (s, 3 H, $CH_3$), 2.01 (s, 6 H, $CH_3$), 2.05 (s, 3 H, $CH_3$), 3.35 (s, 3 H, $OCH_3$), 3.69 (m, 1 H, 5-H), 4.05 (d, d, J = 12 Hz u. 2.5 Hz, 1 H, 6-H), 4.28 (d, d, J = 12 Hz u. 4.5 Hz, 1 H, 6-H), 4.50–5.13 (m, 6 H, 1-H, 1-H, 2-H, 3-H, 4-H) | (KBr): 2990, 2960 2920 (CH), 2840 (Acetal) 1760, 1745 (CO) | C: 48.98 H: 6.17 $C_{16}H_{24}O_{11}$ (392.36) | 49.09 6.19 |
| 1 a + 2 b | A | | 88 | | | | |
| | Z | 1-O-(1'-Methoxy-methyl)-β-D-glucopyranose | | (80 MHz, $D_6$-Aceton/$D_2O$): 3.20–4.00 (m, 6 H, 2-H, 3-H, 4-H, 5-H, 6-H), 3.46 (s, 3 H, $OCH_3$), 4.45 (s, 4 H, OH), 4.58 (d, J = 7.5 Hz, 1 H, 1-H), 4.73 (d, J = 6.5 Hz, 1 H, 1'-H), 5.02 (d, J = 6.5 Hz, 1 H, 1'-H) | | | |
| 1 b + 2 b | A | 1-O-(1'-Methoxy-methyl)-2,3,4,6-tetra-O-acetyl-α-D-glucopyranose (4a) | 87 | (100 MHz, $CDCl_3$): 1.99 (s, 3 H, $CH_3$), 2.01 (s, 3 H, $CH_3$), 2.04 (s, 3 H, $CH_3$), 2.07 (s, 3 H, $CH_3$), 3.38 (s, 3 H, $OCH_3$), 3.99–4.35 (m, 3 H, 5-H, 6-H), 4.54–5.60 (m, 6 H, 1'-H, 1-H, 2-H, 3-H, 4-H) | | | |
| | Z | 1-O-(1'-Methoxy-methyl)-α-D-Glucopyranose | | (80 MHz, $D_6$-Aceton/$D_2O$): 3.50–4.00 (m, 6 H, 2-H, 3-H, 4-H, 5-H, 6-H), 3.55 (s, 3 H, $OCH_3$), 4.20 (s, 4 H, OH), 4.75 (d, J = 6.5 Hz, 1 H, 1'-H), 5.03 (d, J = 6.5 Hz, 1 H, 1'-H), 5.17 (d, J = 3.75 Hz, 1 H, 1-H) | | | |
| 1 a + 2 c | A | 1-O-(1'-Methoxy-2'-phenyl-ethyl)-2,3,4,6-tetra-O-acetyl-β-D-glucopyranose (3b) | 84 | (100 MHz, $CDCl_3$): 2.01 (s, 3 H, $CH_3$), 2.04 (s, 3 H, $CH_3$), 2.06 (s, 3 H, $CH_3$), 2.08 (s, 3 H, $CH_3$), 2.90–3.03 (m, 2 H, 2'-H), 2.35 u. 2.40 (s, 3 H, $OCH_3$), 3.60–3.85 (m, 1 H, 5-H), 4.06–4.33 (m, 2 H, 6-H), 4.71–5.19 (m, 5 H, 1'-H, 1-H, 2-H, 3-H, 4-H), 7.27 (m, 5 H, $C_6H_5$) | (KBr): 3030 (CH arom.) 2980, 2960 2940 (CH aliph) 2840 (Acetal) 1745 (CO) | | |

Fortsetzung

| Edukte | Methode | Produkt | Ausbeute (%) | $^1H$–NMR ($\delta$ = 0 ppm) | IR | Analyse Ber. | Analyse Gef. |
|---|---|---|---|---|---|---|---|
| | Z | 1-O-(1′-Methoxy-2′-phenyl-ethyl)-$\beta$-D-glucopyranose | | (100 MHz, $D_6$-Aceton/$D_2O$): 2.95–3.08 (m, 2 H, 2′-H), 3.32–3.56 (m, 4 H, 2-H, 3-H, 4-H, 5-H), 3.37 u. 3.39 (2 s, 3 H, $OCH_3$), 3.60–3.98 (m, 2 H, 6-H), 4.29 (s, 4 H, OH), 4.57 u. 4.67 (2 d, J = 7.5 Hz, 1 H, 1-H), 4.87 u. 5.03 (2 d, d, J = 7 Hz u. 4 Hz, 1 H, 1′-H), 7.27 (m, 5 H, $C_6H_5$) | | C: 57.32<br>H: 7.06<br>$C_{15}H_{22}O_7$<br>(314.33) | 57.41<br>7.08 |
| 1 a + 2 c | B | 1-O-(1′-Methoxy-2′-phenyl-ethyl)-2,3,4,6-tetra-O-acetyl-$\alpha$-D-glucopyranose (4 b) | 72 | (80 MHz, $CDCl_3$): 2.00 (s, 3 H, $CH_3$), 2.02 (s, 3 H, $CH_3$), 2.04 (s, 3 H, $CH_3$), 2.07 (s, 3 H, $CH_3$), 2.87–3.07 (m, 2 H, 2′-H), 3.35 u. 3.37 (2 s, 3 H, $OCH_3$), 3.55 (d, d, J = 12.5 Hz u. 2.5 Hz, 1 H, 6-H), 3.87 (d, d, J = 12.5 Hz u. 3.75 Hz, 1 H, 6-H), 4.02–4.25 (m, 1 H, 5-H), 4.64–5.63 (m, 5 H, 1′-H, 1-H, 2-H, 3-H, 4-H), 7.25 (m, 5 H, $C_6H_5$) | | | |
| | Z | 1-O-(1′-Methoxy-2′-phenyl-ethyl)-$\alpha$-D-glucopyranose | | (80 MHz, $D_6$-Aceton/$D_2O$): 2.98 (d, J = 5.5 Hz, 2 H, 2′-H), 3.1–3.8 (m, 6 H, 2-H, 3-H, 4-H, 5-H, 6-H), 3.37 u. 3.39 (2 s, 3 H, $OCH_3$), 3.89 (s, 4 H, OH), 4.91 (t, J = 5.5 Hz, 1 H, 1′-H), 5.09 (d, J = 3.75 Hz, 1 H, 1-H), 7.25 (m, 5 H, $C_6H_5$) | | | |
| 1 a + 2 d | A | 1-O-(1′-Methoxy-butyl)-2,3,4,6-tetra-O-acetyl-$\beta$-D-glucopyranose (3 c) | 78 | (100 MHz, $CDCl_3$): 0.95 (t, J = 7 Hz, 3 H, 4′-H), 1.22–1.82 (m, 4 H, 2′-H, 3′-H), 2.01 (s, 3 H, $CH_3$), 2.04 (s, 6 H, $CH_3$), 2.08 (s, 3 H, $CH_3$), 3.31 u. 3.37 (2 s, 3 H, $OCH_3$), 3.62–3.80 (m, 1 H, 5-H), 4.08–4.34 (m, 2 H, 6-H), 4.49–5.34 (m, 5 H, 1′-H, 1-H, 2-H, 3-H, 4-H) | (Film)<br>2960, 2940<br>2880 (CH)<br>2840 (Acetal)<br>1745 (CO) | C: 52.53<br>H: 6.96<br>$C_{19}H_{30}O_{11}$<br>(434.44) | 52.60<br>7.01 |

Fortsetzung

| Edukte | Methode | Produkt | Ausbeute (%) | $^1$H—NMR ($\delta = 0$ ppm) | IR | Analyse Ber. | Analyse Gef. |
|---|---|---|---|---|---|---|---|
| | Z | 1-O-(1'-Methoxy-butyl)-$\beta$-D-glucopyranose | | (100 MHz, $D_6$-Aceton/$D_2O$): 0.89 (t, J = 7.0 Hz, 3 H, 4'-H), 1.14—1.80 (m, 4 H, 2'-H, 3'-H), 3.18—3.54 (m, 4 H, 2-H, 3-H, 4-H, 5-H), 3.38 u. 3.42 (2 s, 3 H, $OCH_3$), 3.66 (d, d, J = 12.5 Hz u. 2.5 Hz, 1 H, 6-H), 3.86 (d, d, J = 12.0 Hz u. 2.0 Hz, 1 H, 6-H), 4.05 (s, 4 H, OH), 4.47 u. 4.59 (2 d, J = 7.5 Hz, 1 H, 1-H), 4.64 u. 4.83 (2 t, J = 5.0 Hz, 1 H, 1'-H) | | | |
| 1 a + 2 d | B | 1-O-(1'-Methoxy-butyl)-2,3,4,6-tetra-O-acetyl-$\alpha$-D-glucopyranose (4c) | 57 | (60 MHz, $CDCl_3$): 0.80—1.60 (m, 7 H, 2'-H, 3'-H, 4'-H), 1.92 (s, 3 H, $CH_3$), 1.94 (s, 3 H, $CH_3$), 1.96 (s, 3 H, $CH_3$), 2.00 (s, 3 H, $CH_3$), 3.23 u. 3.34 (2 s, 3 H, $OCH_3$), 3.89—4.26 (m, 3 H, 5-H, 6-H), 4.45—5.60 (m, 5 H, 1'-H, 1-H, 2-H, 3-H, 4-H) | | | |
| | Z | 1-O-(1'-Methoxy-butyl)-$\alpha$-D-glucopyranose | | (80 MHz, $D_6$-Aceton/$D_2O$): 0.82—1.82 (m, 7 H, 2'-H, 3'-H, 4'-H), 3.36 u. 3.42 (2 s, 3 H, $OCH_3$), 3.28—3.85 (m, 6 H, 2-H, 3-H, 4-H, 5-H, 6-H), 4.01 (s, 4 H, OH), 4.53—4.90 (m, 1 H, 1'-H), 5.02 u. 5.08 (2 d, J = 3.75 Hz, 1 H, 1-H) | | | |
| 1 a + 2 e | A | 1-O-(1',3',3'-Trimethoxy-propyl)-2,3,4,6-tetra-O-acetyl-$\beta$-D-glucopyranose | 79 | (60 MHz, $CDCl_3$): 2.00 (s, 3 H, $CH_3$), 2.02 (s, 6 H, $CH_3$), 2.04 (s, 3 H, $CH_3$), 1.83—2.04 (m, 2 H, 2'-H), 3.29 u. 3.36 (2 s, 3 H, $OCH_3$), 3.30 (s, 6 H, $OCH_3$), 3.55—3.86 (m, 1 H, 5-H), 3.92—4.28 (m, 2 H, 6-H), 4.34—5.18 (m, 6 H, 1-H, 1'-H, 2-H, 3-H, 3'-H, 4-H) | (Film): 2950, 2920 (CH) 2850 (Acetal) 1750 (CO) | | |
| | Z | 1-O-(1',3',3'-Trimethoxy-propyl)-$\beta$-D-glucopyranose | | (80 MHz, $D_6$-Aceton/$D_2O$): 1.89-2.14 (m, 2 H, 2'-H), 3.33 (s, 6 H, $OCH_3$), 3.43 u. 3.45 (2 s, 3 H, $OCH_3$), 3.16—3.95 (m, 6 H, 2-H, 3-H, 4-H, 5-H, 6-H), 4.12 (s, 4 H, OH), 4.63 u. 4.71 (2 d, J = 7.5 Hz, 1 H, 1-H), darunter 4.45—4.93 (m, 2 H, 1'-H, 3'-H) | | | |

Fortsetzung

| Edukte | Methode | Produkt | Ausbeute (%) | $^1$H—NMR ($\delta = 0$ ppm) | IR | Analyse Ber. | Analyse Gef. |
|---|---|---|---|---|---|---|---|
| 1a + 2e | B | 1-O-(1',3',3'-Trimethoxypropyl)-2,3,4,6-tetra-O-acetyl-$\alpha$-D-glucopyranose (4d) | 79 | (100 MHz, $CDCl_3$): 1.92—2.14 (m, 2H, 2'-H), 1.98 (s, 3H, $CH_3$), 2.00 (s, 3H, $CH_3$), 2.02 (s, 3H, $CH_3$), 2.06 (s, 3H, $CH_3$), 3.26 u. 3.38 (2s, 3H, $OCH_3$), 3.30 (s, 6H, $OCH_3$), 3.99—4.26 (m, 3H, 5-H, 6-H), 4.39—5.57 (m, 6H, 1-H, 1'-H, 2-H, 3-H, 3'-H, 4-H) | | | |
| | Z | 1-O-(1',3',3'-Trimethoxypropyl)-$\alpha$-D-glucopyranose | | (100 MHz, $D_6$-Aceton/$D_2O$): 1.92—2.17 (m, 2H, 2'-H), 3.32 (s, 6H, $OCH_3$), 3.38 u. 3.42 (2s, 3H, $OCH_3$), darunter bis 3.82 (m, 6H, 2-H, 3-H, 4-H, 5-H, 6-H), 4.26 (s, 4H, OH), 4.49—4.88 (m, 2H, 1'-H, 3'-H), 5.02 u. 5.12 (2d, J = 3.75 Hz, 1H, 1-H) | | | |
| 1a + 2f | A | 1-O-(1',2'-Dimethoxyethyl)-2,3,4,6-tetra-O-acetyl-$\beta$-D-glucopyranose (3e) | 77 | (60 MHz, $CDCl_3$): 2.00 (s, 3H, $CH_3$), 2.02 (s, 3H, $CH_3$), 2.03 (s, 3H, $CH_3$), 2.04 (s, 3H, $CH_3$), 3.33—3.47 (m, 8H, 2'-H, $OCH_3$), 3.56—3.89 (m, 1H, 5-H), 4.07—4.36 (m, 2H, 6-H), 4.70—5.21 (m, 5H, 1-H, 1'-H, 2-H, 3-H, 4-H) | | | |
| | Z | 1-O-(1',2'-Dimethoxyethyl)-$\beta$-D-glucopyranose | | (100 MHz, $D_6$-Aceton/$D_2O$): 3.21—3.95 (m, 8H, 2-H, 2'-H, 3-H, 4-H, 5-H, 6-H), 3.35 (s, 3H, $OCH_3$), 3.45 u. 4.47 (2s, 3H, $OCH_3$), 4.18 (s, 4H, OH), 4.57 u. 4.67 (2d, J = 7.5 Hz, 1H, 1-H), 4.79 u. 4.94 (2t, J = 5.5 Hz, 1H, 1'-H) | | | |
| 1a + 2f | B | 1-O-(1',2'-Dimethoxyethyl)-2,3,4,6-tetra-O-acetyl-$\alpha$-D-glucopyranose (4e) | 84 | (100 MHz, $CDCl_3$): 2.02 (s, 3H, $CH_3$), 2.04 (s, 3H, $CH_3$), 2.06 (s, 3H, $CH_3$), 2.10 (s, 3H, $CH_3$), 3.36, 3.40 u. 3.47 (3s, 6H, $OCH_3$), 3.30—3.52 (m, 2H, 2'-H), 3.98—4.37 (m, 3H, 5-H, 6-H), 4.64—5.64 (m, 5H, 1-H, 1'-H, 2-H, 3-H, 4-H) | (Film): 3020, 2940 (CH) 2850 (Acetal) 1750 (CO) | | |

Fortsetzung

| Edukte | Methode | Produkt | Ausbeute (%) | $^1$H–NMR ($\delta$ = 0 ppm) | IR | Analyse Ber. | Analyse Gef. |
|---|---|---|---|---|---|---|---|
| | Z | 1-O-(1',2'-Dimethoxyethyl)-$\alpha$-D-glucopyranose | | (80 MHz, $D_6$-Aceton/$D_2O$): 3.40–3.78 (m, 8 H, 2'-H, 2-H, 3-H, 4-H, 5-H, 6-H), 3.40, 3.48 u. 3.53 (3 s, 6 H, $OCH_3$), 4.52 (s, 4 H, OH), 4.72–4.96 (m, 1 H, 1'-H), 5.12 u. 5.20 (2 d, J = 3.75 Hz, 1 H, 1-H) | | | |
| 1a + 2g | A | 1-O-(2'-Chlor-1'-methoxy-ethyl)-2,3,4,6-tetra-O-acetyl-$\beta$-D-glucopyranose | 71 | (60 MHz, $CDCl_3$): 2.03 (s, 3 H, $CH_3$), 2.06 (s, 6 H, $CH_3$), 2.08 (s, 3 H, $CH_3$), 3.45–3.98 (m, 6 H, 2'-H, 5-H, $OCH_3$), 4.20–4.34 (m, 2 H, 6-H), 4.67–5.23 (m, 5 H, 1-H, 1'-H, 2-H, 3-H, 4-H) | | | |
| | Z | 1-O-(2'-Chlor-1'-methoxy-ethyl)-$\beta$-D-glucopyranose | | (80 MHz, $D_6$-Aceton/$D_2O$): 3.40–3.95 (m, 8 H, 2'-H, 2-H, 3-H, 4-H, 5-H, 6-H), 3.42 u. 3.49 (2 s, 3 H, $OCH_3$), 3.65 (s, 4 H, OH), 4.56 u. 4.62 (2 d, J = 7.5 Hz, 1 H, 1-H), 4.82 u. 4.92 (2 d, d, J = 7 Hz u. 3.75 Hz, 1 H, 1'-H) | | | |
| 1a + 2g | B | 1-O-(2'-Chlor-1'-methoxy-ethyl)-2,3,4,6-tetra-O-acetyl-$\alpha$-D-glucopyranose (4f) | 82 | (100 MHz, $CDCl_3$): 1.98 (s, 3 H, $CH_3$), 2.01 (s, 3 H, $CH_3$), 2.02 (s, 3 H, $CH_3$), 2.05 (s, 3 H, $CH_3$), 3.37–3.59 (m, 5 H, 2'-H, $OCH_3$), 4.01–4.29 (m, 3 H, 5-H, 6-H), 4.62–5.58 (m, 5 H, 1-H, 1'-H, 2-H, 3-H, 4-H) | (Film): 2980, 2950 (CH) 2860 (Acetal) 1750 (CO) | | |
| | Z | 1-O-(2'-Chlor-1'-methoxy-ethyl)-$\alpha$-D-glucopyranose | | (60 MHz, $D_6$-Aceton/$D_2O$): 3.33–3.86 (m, 8 H, 2-H, 2'-H, 3-H, 4-H, 5-H, 6-H), 3.50 u. 3.53 (2 s, 3 H, $OCH_3$), 4.25 (s, 4 H, OH), 4.56–5.01 (m, 1 H, 1'-H), 5.19 u. 5.21 (2 d, J = 3.75 Hz, 1 H, 1-H) | | | |
| 1a + 2h | A | 1-O-(2'-Brom-1'-methoxy-ethyl)-2,3,4,6-tetra-O-acetyl-$\beta$-D-glucopyranose (3g) | 78 | (60 MHz, $CDCl_3$): 2.02 (s, 3 H, $CH_3$), 2.04 (s, 3 H, $CH_3$), 2.06 (s, 3 H, $CH_3$), 2.10 (s, 3 H, $CH_3$), 3.36–3.50 (m, 5 H, 2'-H, $OCH_3$), 3.67–3.93 (m, 1 H, 5-H), 4.04–4.36 (m, 2 H, 6-H), 4.73–5.26 (m, 5 H, 1-H, 1'-H, 2-H, 3-H, 4-H) | | | |

Fortsetzung

| Edukte | Methode | Produkt | Ausbeute (%) | $^1$H—NMR ($\delta = 0$ ppm) | IR | Analyse Ber. | Analyse Gef. |
|---|---|---|---|---|---|---|---|
| | Z | 1-O-(2′-Brom-1′-methoxy-ethyl)-β-D-glucopyranose | | (80 MHz, $D_6$-Aceton/$D_2O$): 3.30—3.95 (m, 11 H, 2′-H, $OCH_3$, 2-H, 3-H, 4-H, 5-H, 6-H), 4.11 (s, 4 H, OH), 4.63 u. 4.71 (2 d, J = 7.5 Hz, 1 H, 1-H), 4.79—5.00 (m, 1 H, 1′-H) | | | |
| 1a + 2h | B | 1-O-(2′-Brom-1′-methoxy-ethyl)-2,3,4,6-tetra-O-acetyl-α-D-glucopyranose (4g) | 83 | (100 MHz, $CCl_4$): 2.00 (s, 3 H, $CH_3$), 2.02 (s, 3 H, $CH_3$), 2.04 (s, 3 H, $CH_3$), 2.07 (s, 3 H, $CH_3$), 3.38 u. 3.46 (2 s, 3 H, $OCH_3$), 3.28—3.52 (m, 2 H, 2′-H), 3.94—4.35 (m, 3 H, 5-H, 6-H), 4.65—5.60 (m, 5 H, 1-H, 1′-H, 2-H, 3-H, 4-H) | (Film): 2960 (CH) 2840 (Acetal) 1745 (CO) | C: 42.08 H: 5.19 $C_{17}H_{25}O_{11}Br$ (485.29) | 41.95 5.16 |
| | Z | 1-O-(2′-Brom-1′-methoxy-ethyl)-α-D-glucopyranose | | (200 MHz, $D_6$-Aceton/$D_2O$): 3.42—3.88 (m, 8 H, 2-H, 2′-H, 3-H, 4-H, 5-H, 6-H), 3.50 u. 3.54 (2 s, 3 H, $OCH_3$), 4.53 (s, 4 H, OH), 4.89 u. 4.95 (2 d, d, J = 5.5 Hz u. 4.0 Hz, 1 H, 1′-H), 5.19 u. 5.21 (2 d, J = 3.75 Hz, 1 H, 1-H) | | | |
| 1a + 2i | | 1-O-(1′-Dimethoxy-methyl)-6-O-methyl-2,3,4-tetra-O-acetyl-β-D-glucopyranose (6) | | Massenspektrum: MS(M/e): 363 (0.4, $M^+ - CH_3O$), 319 (0,4, $M^+ - C_3H_7O_2$), 303 (1.3, $M^+ - C_3H_7O_3$), 243 (4, 303 $- C_2H_4O_2$), 183 (4, 243 $- C_2H_4O_2$), 141 (14, 183 $- C_2H_2O$), 75 (98, $C_3H_7O_2^+$) | | | |
| 1c + 2b | A | 1-O-(1′-Methoxy-methyl)-2,3,4,6-tetra-O-benzyl-β-D-glucopyranose (6a) | 89 | (60 MHz, $CCl_4$): 3.34 (s, 3 H, $OCH_3$), 3.42—3.98 (m, 6 H, 2-H, 3-H, 4-H, 5-H, 6-H), 4.41—4.92 (m, 11 H, 1-H, 1′-H, $CH_2$), 7.04—7.33 (m, 20 H, $C_6H_5$) | (Film): 3105, 3080 3050 (CH arom) 2940, 2910 2880 (CH aliph) 2840 (Acetal) 1620, 1590, 1500 (Aromat) 740, 700 (Benzol-ring, monosubst.) | | |
| 1d + 2b | A | 1-O-(1′-Methoxy-methyl)-2,3,4,6-tetra-O-benzyl-α-D-glucopyranose (6b) | | (100 MHz, $CDCl_3$): 3.37 (s, 3 H, $OCH_3$), 3.52—4.12 (m, 6 H, 2-H, 3-H, 4-H, 5-H, 6-H), 4.34—5.03 (m, 10 H, 1′-H, $CH_2$), 5.13 (d, J = 3.75 Hz, 1 H, 1-H), 7.04—7.33 (m, 20 H, $C_6H_5$) | | | |

Fortsetzung

| Edukte | Methode | Produkt | Ausbeute (%) | $^1$H—NMR ($\delta$ = 0 ppm) | IR | Analyse Ber. | Gef. |
|---|---|---|---|---|---|---|---|
| 7 + 2b | A | 1-$\alpha$-O-(1'-Methoxy-methyl)-1,4a$\alpha$,5,6,7a$\alpha$-hexahydro-cyclopenta(c)pyran-4-carbonsäuremethylester (8) | 90 | (100 MHz, $CDCl_3$): 1.18–2.32 (m, 7H, 5-H, 6-H, 7a-H), 2.76 (q, J = 8.0 Hz, 1H, 4a-H), 3.40 (s, 3H, $OCH_3$), 3.69 (s, 3H, $CH_3$), 4.59 (d, J = 6.5 Hz, 1H, 1'-H), 4.76 (d, J = 7.5 Hz, 1H, 1-H), 4.97 (d, J = 6.5 Hz, 1H, 1'-H), 7.39 (d, J = 1.0 Hz, 1H, 3-H) | (Film): 2960, 2900 2880 (CH) 2935 (Acetal) 1610 (CO) 1630 (C = C) | 242.1154 | 242.1155 (Massenspektr.) $C_{12}H_{18}O_5$ |

**Patentansprüche**

1. Verfahren zur Herstellung von 1,1'-Diacetalen der allgemeinen Formel I

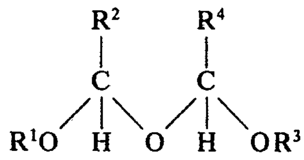

(I)

in der

$R_1$ und $R_2$ die gleich oder verschieden sein können, einen gegebenenfalls substituierten aliphatischen gesättigten oder ungesättigten Kohlenwasserstoffrest darstellen, wobei $R_1$ und $R_2$ miteinander verbunden sein können
$R_3$ gegebenenfalls substituiertes Alkyl und
$R_4$ gegebenenfalls substituiertes O-Alkyl oder einen gegebenenfalls substituierten gesättigten oder ungesättigten aliphatischen Rest darstellen, wobei $R_3$ und $R_4$ miteinander verbunden sein können,

dadurch gekennzeichnet, daß man eine Silylverbindung der allgemeinen Formel II

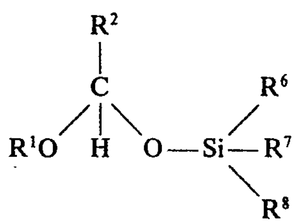

(II)

in der

$R_1$ und $R_2$ die obengenannte Bedeutung haben und in der
$R_6$, $R_7$ und $R_8$ die gleich oder verschieden sein können einen gegebenenfalls substituierten Alkylrest darstellen

mit einem Acetal der allgemeinen Formel III

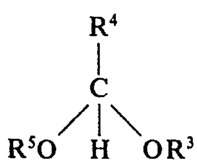

(III)

in der

$R_3$ und $R_4$ die obengenannte Bedeutung haben und
$R_5$ gegebenenfalls substituiertes Alkyl oder Phenyl darstellt

in Gegenwart eines Katalysators der allgemeinen Formel IV oder V

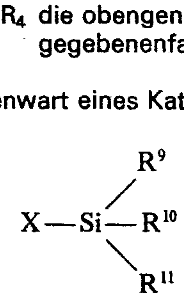

(IV)

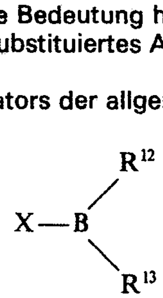

(V)

in denen

$R_9$, $R_{10}$, $R_{11}$, $R_{12}$ und $R_{13}$ die gleich oder verschieden sein können einen Alkylrest mit 1–8 C-Atomen darstellen und

X für $FSO_3$, $CF_3CO_2$, $ClO_4$, $BF_4$, $CH_3SO_3$ oder $CF_3(CF_2)_n-SO_3$ mit n = 0–4 steht

in einem geeigneten Lösungsmittel umsetzt.

2. Verfahren nach Anspruch 1, dadurch gekennzeichnet, daß die Umsetzung zwischen −80°C und 100°C durchgeführt wird.

3. Verfahren nach den Ansprüchen 1–2, dadurch gekennzeichnet, daß die Umsetzung in $CH_2Cl_2$, $CHCl_3$, Acetonitril, Diethylether oder Tetrahydrofuran durchgeführt wird.

4. Verfahren nach den Ansprüchen 1–3, dadurch gekennzeichnet, daß die Silylverbindung II mit einer äquimolaren Menge oder einem Überschuß an Acetal III umgesetzt wird.

5. Verfahren nach den Ansprüchen 1–4, dadurch gekennzeichnet, daß etwa 1/10 Äquivalente, bezogen auf die Silylverbindung II an Katalysator IV oder V zugegeben wird.

6. Verfahren nach den Ansprüchen 1–5, dadurch gekennzeichnet, daß X für $CF_3SO_3$ steht.

7. Verfahren nach den Ansprüchen 1–6, dadurch gekennzeichnet, daß $R_6$, $R_7$, $R_8$, $R_9$, $R_{10}$, $R_{11}$, $R_{12}$ und $R_{13}$ Methyl bedeuten.

## Claims

1. Process for the preparation of 1,1'-diacetals of the general formula I

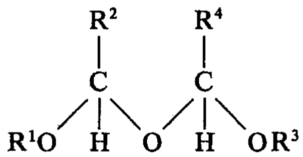

(I)

in which

$R_1$ and $R_2$, which can be identical or different, represent an optionally substituted aliphatic saturated or unsaturated hydrocarbon radical, it being possible for $R_1$ and $R_2$ to be bonded to one another,

$R_3$ represents optionally substituted alkyl and

$R_4$ represents optionally substituted O-alkyl or an optionally substituted saturated or unsaturated aliphatic radical, it being possible for $R_3$ and $R_4$ to be bonded to one another,

characterised in that a silyl compound of the general formula II

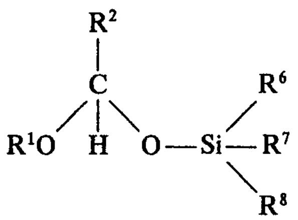

(II)

in which

$R_1$ and $R_2$ have the abovementioned meaning and in which

$R_6$, $R_7$ and $R_8$, which can be identical or different, represent an optionally substituted alkyl radical,

is reacted with an acetal of the general formula III

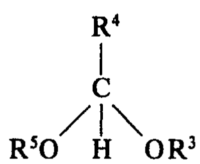

(III)

in which

$R_3$ and $R_4$ have the abovementioned meaning and

$R_5$ represents optionally substituted alkyl or phenyl

in the presence of a catalyst of the general formula IV or V

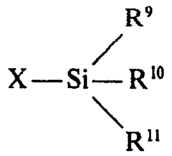 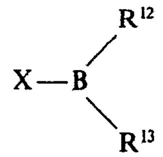

(IV) (V)

in which

$R_9$, $R_{10}$, $R_{11}$, $R_{12}$ and $R_{13}$, which can be identical or different, represent an alkyl radical with 1–8 C atoms and

X represents $FSO_3$, $CF_3CO_2$, $ClO_4$, $BF_4$, $CH_3SO_3$ or $CF_3(CF_2)_n-SO_3$ in which n = 0–4,

in a suitable solvent.

2. Process according to Claim 1, characterised in that the reaction is carried out at between −80°C and 100°C.

3. Process according to Claims 1–2, characterised in that the reaction is carried out in $CH_2Cl_2$, $CHCl_3$, acetonitrile, diethyl ether or tetrahydrofuran.

4. Process according to Claims 1–3, characterised in that the silyl compound II is reacted with an equimolar amount or an excess of the acetal III.

5. Process according to Claims 1–4, characterised in that about 1/10 equivalent, based on the silyl compound II, of catalyst IV or V is added.

6. Process according to Claims 1–5, characterised in that X represents $CF_3SO_3$.

7. Process according to Claims 1–6, characterised in that $R_6$, $R_7$, $R_8$, $R_9$, $R_{10}$, $R_{11}$, $R_{12}$ and $R_{13}$ denote methyl.

**Revendications**

1. Procédé de production de 1,1'-diacétals de formule générale I

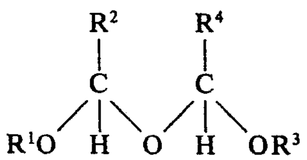

(I)

dans laquelle

$R_1$ et $R_2$, qui peuvent être égaux ou différents, représentent un reste hydrocarboné aliphatique saturé ou insaturé éventuellement substitué, $R_1$ et $R_2$ pouvant être liés ensemble

$R_3$ est un groupe éventuellement substitué et

$R_4$ est un groupe O-alkyle éventuellement substitué ou un reste aliphatique saturé ou insaturé éventuellement substitué, $R_3$ et $R_4$ pouvant être liés ensemble,

caractérisé en ce qu'on fait réagir dans un solvant approprié un composé silylique de formule générale II

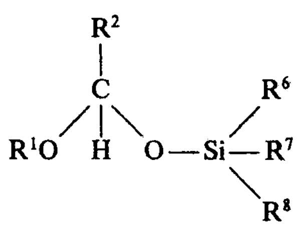

(II)

dans laquelle

$R_1$ et $R_2$ ont la définition indiquée ci-dessus et

$R_6$, $R_7$ et $R_8$ peuvent être égaux ou différents et représentent un reste alkyle éventuellement substitué

avec un acétal de formule générale III

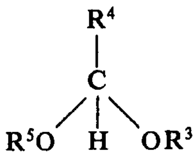

(III)

dans laquelle

$R_3$ et $R_4$ ont la définition indiquée ci-dessus et
$R_5$ représente un groupe alkyle ou phényle éventuellement substitué

en présence d'un catalyseur de formule générale IV ou V

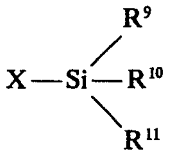

(IV)

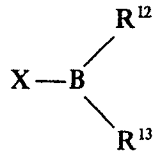

(V)

formules dans lesquelles

$R_9$, $R_{10}$, $R_{11}$, $R_{12}$ et $R_{13}$, qui peuvent être égaux ou différents, représentent un reste alkyle ayant 1 à 8 atomes de carbone et
X représente $FSO_3$, $CF_3CO_2$, $ClO_4$, $BF_4$, $CH_3SO_3$ ou $CF_3(CF_2)_n-SO_3$, avec $n = 0-4$.

2. Procédé suivant la revendication 1, caractérisé en ce que la réaction est conduite entre −80°C et 100°C.
3. Procédé suivant les revendications 1 et 2, caractérisé en ce que la réaction est conduite dans $CH_2Cl_2$, $CHCl_3$, l'acétonitrile, l'ether diéthylique ou le tétrahydrofuranne.
4. Procédé suivant les revendications 1–3, caractérisé en ce que le composé de silyle II est amené à réagir avec une quantité équimolaire ou un excès d'acétal III.
5. Procédé suivant les revendications 1–4, caractérisé en ce qu'on ajoute environ 1/10 équivalent, par rapport au composé silylique II, de catalyseur IV ou V.
6. Procédé suivant les revendications 1–5, caractérisé en ce que X représente $CF_3SO_3$.
7. Procédé suivant les revendications 1–6, caractérisé en ce que $R_6$, $R_7$, $R_8$, $R_9$, $R_{10}$, $R_{11}$, $R_{12}$ et $R_{13}$ désignent des groupes méthyle.